(19) Europäisches Patentamt
European Patent Office
Office européen des brevets

(11) EP 4 541 371 A1

(12) # EUROPEAN PATENT APPLICATION
published in accordance with Art. 153(4) EPC

(43) Date of publication:
**23.04.2025 Bulletin 2025/17**

(21) Application number: **23823229.2**

(22) Date of filing: **15.06.2023**

(51) International Patent Classification (IPC):
***A61K 39/395*** *(2006.01)* ***A61K 47/68*** *(2017.01)*
***A61K 9/19*** *(2006.01)* ***A61P 35/00*** *(2006.01)*

(52) Cooperative Patent Classification (CPC):
**A61K 9/19; A61K 39/395; A61K 47/68; A61P 35/00**

(86) International application number:
**PCT/CN2023/100483**

(87) International publication number:
**WO 2023/241663 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.06.2022 CN 202210679133**

(71) Applicants:
- **Shanghai Hansoh Biomedical Co., Ltd.**
  **Shanghai 201203 (CN)**
- **Changzhou Hansoh Pharmaceutical Co., Ltd.**
  **Xinbei District**
  **Changzhou**
  **Jiangsu 213001 (CN)**
- **Jiangsu Hansoh Pharmaceutical Group Co., Ltd.**
  **Lianyungang, Jiangsu 222047 (CN)**

(72) Inventor: **MAO, Dongjie**
**Shanghai 201203 (CN)**

(74) Representative: **Wilkinson, Johanna Elise**
**GSK**
**Legal & Compliance - Global Patents**
**79 New Oxford Street**
**London WC1A 1DG (GB)**

(54) **PHARMACEUTICAL COMPOSITION COMPRISING ANTIBODY-DRUG CONJUGATE AND USE THEREOF**

(57) The present disclosure relates to a pharmaceutical composition containing an antibody-drug conjugate and its use. Specifically, the present disclosure relates to a pharmaceutical composition that contains an antibody-drug conjugate in a buffer solution. The pharmaceutical composition involved in the present disclosure exhibits good stability.

EP 4 541 371 A1

## Description

## Technical Field

The present disclosure belongs to the field of pharmaceutical formulation, and specifically relates to a pharmaceutical composition containing an antibody-drug conjugate and its use as an anticancer drug.

## Background Art

The statements here only provide background information related to the present disclosure and do not necessarily constitute prior art.

An antibody-drug conjugate (ADC) links monoclonal antibody or antibody fragments to bioactive cytotoxins through stable chemical linker compounds, fully leveraging the specificity of antibodies for tumour cell surface antigens and the high efficiency of cytotoxins, while avoiding the deficiencies including low efficacy of the former and excessive toxic and side effects of the latter. This means that, compared with conventional chemotherapy drugs, antibody-drug conjugates can precisely target tumour cells while minimising the effect on normal cells (Mullard A, (2013) Nature Reviews Drug Discovery, 12:329-332; DiJoseph JF, Armellino DC, (2004) Blood, 103:1807-1814).

There are several classes of cytotoxic small molecules used for antibody-drug conjugates, one of which is camptothecin derivatives, which exert their antitumour effects by inhibiting topoisomerase I. The literature, in which the application of camptothecin derivative Exatecan (chemical name:(1S, 9S)-1-amino-9-ethyl-5-fluoro-2,3-dihydro-9-hydroxy-4-methyl-1H, 12H-benzo [de] pyrano [3, 4,: 6,7] imidazo [1,2-b] quinoline-10, 13 (9H, 15H)-dione) in antibody-drug conjugates (ADCs) was reported, includes WO2014057687; Clinical Cancer Research (2016) 22(20):5097-5108; Cancer Sci (2016) 107: 1039-1046。However, further development of more effective ADC drugs is still needed.

Moreover, ADCs have more complex heterogeneous structures than antibodies, thus posing greater challenges for ADC formulations intended for therapeutic purposes.

## Summary of Invention

The present disclosure describes a pharmaceutical composition comprising an antibody-drug conjugate and a buffer, where the antibody-drug conjugate has the structure as shown below:

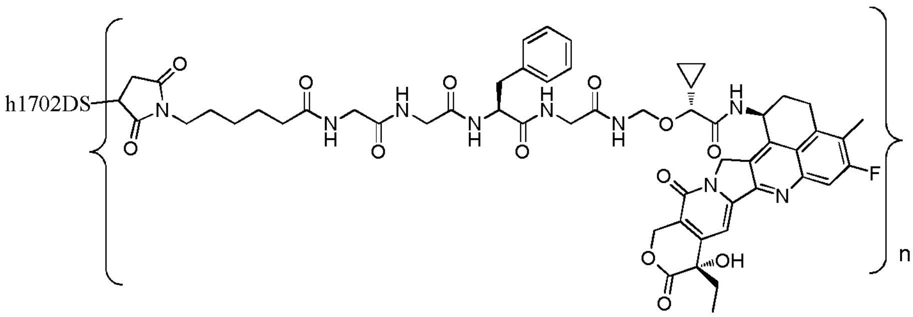

;

The antibody-drug conjugate as described above is prepared by reference to the preparation of FADC-2 in Example 9 of antibody conjugate on Pages 45 - 47 in the description of WO2020063673A1;

Wherein: h1702DS is an anti-B7H3 antibody (the source of which refers to the antibody h1702DS on Page 44 of WO2020063673A1), containing the heavy chain as shown in SEQ ID NO:1 (SEQ ID NO:14 in WO2020063673A1) and the light chain as shown in SEQ ID NO:2 (SEQ ID NO:16 in WO2020063673A1):

The amino acid sequence of the heavy chain of h1702DS:(SEQ ID NO:1)

QVQLVQSGGGVVQPGTSLRLSCAASGFIFSSSAMHWVRQAPGKGLEWVAV
ISYDGSNKYYVDSVKGRFTISRDNSKNTLYLQMNSLRAEDTAVYYCARSARLYA
SFDYWGQGALVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK

The amino acid sequence of the light chain of h1702DS:(SEQ ID NO:2)

DTVVTQEPSFSVSPGGTVTLTCGLSSGSVSTSHYPSWYQQTPGQAPRMLIYN
TNTRSSGVPDRFSGSILGNKAALTITGAQADDESDYYCAIHVDRDIWVFGGGTK
LTVLGQPKANPTVTLFPPSSEELQANKATLVCLISDFYPGAVTVAWKADGSPVKA
GVETTKPSKQSNNKYAASSYLSLTPEQWKSHRSYSCQVTHEGSTVEKTVAPTEC;

n ranges from 1 to 10, preferably 1 to 8, more preferably 3 to 5, and further more preferably about 4;

The buffer of the pharmaceutical composition is succinate buffer or histidine buffer, preferably succinic acid-sodium succinate, histidine-acetate or histidine-hydrochloride buffer.

The concentration of the buffer of the pharmaceutical composition is about 15 mM to about 50 mM, preferably about 20 mM to about 40 mM, and more preferably about 30 mM.

The pH of the pharmaceutical composition is about 4.5 to about 6.5, preferably about 5.0 to about 6.0, and more preferably about 5.5 to about 5.6.

In optional embodiments, the pH of the buffer in the pharmaceutical composition is about 4.5 to about 6.5, with non-limiting examples including about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5 and any range between these point values, preferably about 5.0 to about 6.0, and more preferably about 5.5 to about 5.6.

In optional embodiments, the succinate buffer in the pharmaceutical composition is succinic acid-sodium succinate, and the histidine buffer is preferably histidine-acetate or histidine-hydrochloride buffer.

In optional embodiments, the pharmaceutical composition also contains a surfactant. The surfactant can be polysorbate, polysorbate 20, polysorbate 80, poloxamer, triton, sodium dodecyl sulfonate, sodium lauryl sulfonate, sodium caprylglycoside, laurylsulphobetaine, myristyl-sulphobetaine, linoleic-sulphobetaine, stearyl-sulphobetaine, lauryl-sarcosine, myristyl-sarcosine, linoleic-sarcosine, stearyl-sarcosine, linoleicbetaine, myristyl-betaine, cetyl-betaine, laurylamide propyl betaine, cocamidopropyl betaine, linoleamidopropyl betaine, myristamidopropyl betaine, palmitamidopropyl betaine, isostearamidopropyl betaine, myristamidopropyl dimethylamine, palmitamidopropyl dimethylamine, isostearamidopropyl dimethylamine, sodium methyl cocoyl sarcosinate, sodium methyl oleoyl sarcosinate, polyethylene glycol, polypropylene glycol, copolymers of ethylene and propylene glycol, etc. The preferred surfactant is polysorbate 80 or polysorbate 20, and more preferably polysorbate 80.

In optional embodiments, the concentration of the surfactant in the pharmaceutical composition is about 0.01 mg/mL to about 1.0 mg/mL, preferably about 0.05 mg/mL to about 0.5 mg/mL, more preferably about 0.1 mg/mL to about 0.4 mg/mL or about 0.2 mg/mL to about 0.3 mg/mL, and most preferably about 0.2 mg/mL, with non-limiting examples including 0.02 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.8 mg/mL, and any range between these point values.

In optional embodiments, the aforementioned pharmaceutical composition also contains sugars. The "sugars" involved in the present disclosure include conventional compositions $(CH_2O)_n$ and their derivatives, such as monosaccharides, disaccharides, trisaccharides, polysaccharides, sugar alcohols, reducing sugars and non-reducing sugars. The sugars can be glucose, sucrose, trehalose, lactose, fructose, maltose, dextran, glycerin, erythritol, glycerol, arabitol, sylitol, sorbitol, mannitol, melibiose, melezitose, melitriose, manninotriose, stachyose, maltose, lactulose, maltulose,

sorbitol, maltitol, lactitol, isomaltulose, etc. The preferred sugars are non-reducing disaccharides, more preferably trehalose, mannitol or sucrose, and most preferably sucrose or trehalose.

In optional embodiments, the concentration of sugar in the aforementioned pharmaceutical composition is about 25 mg/mL to about 80 mg/mL, preferably about 30 mg/mL to about 60 mg/mL, and more preferably about 40 mg/mL, with non-limiting examples including 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, and any range between these point values, preferably 40 mg/mL.

In optional embodiments, the aforementioned pharmaceutical composition also contains amino acids and their salts, which are preferably glycine and arginine hydrochloride, and more preferably glycine.

In optional embodiments, the concentration of the glycine in the aforementioned pharmaceutical composition is about 5 mg/mL to about 10 mg/mL, about 5.3 mg/mL to about 9.8 mg/mL, and about 6 mg/mL to about 9 mg/mL, preferably about 7 mg/mL to about 8 mg/mL, with non-limiting examples including 6 mg/mL, 6.5 mg/mL, 7 mg/mL, 7.2 mg/mL, 7.6 mg/mL, 7.8 mg/mL, 8 mg/mL, 8.5 mg/mL, 9 mg/mL, and any range between these point values, most preferably about 7.6 mg/mL.

In optional embodiments, the aforementioned pharmaceutical composition further contains arginine hydrochloride, which is at a concentration of about 40 mM to about 80 mM, preferably about 50 mM to about 70 mM, with non-limiting examples including 40 mM, 45 mM, 50 mM, 55 mM, 58 mM, 60 mM, 65 mM, 70 mM, 75 mM and 80 mM, most preferably about 58 mM.

In optional embodiments, the antibody-drug conjugate in the pharmaceutical composition is at a protein concentration of about 1 mg/mL to about 100 mg/mL, with non-limiting examples including 1 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, 40 mg/mL, 50 mg/mL, 60 mg/mL, 70 mg/mL, 80 mg/mL, 90 mg/mL, 100 mg/mL and any range between these point values, preferably about 10 mg/mL to about 70 mg/mL, more preferably about 20 mg/mL to about 50 mg/mL, further preferably about 20 mg/mL to about 30 mg/mL or about 30 mg/mL to about 50 mg/mL, and even further preferably about 20 mg/mL or about 30 mg/mL or about 50 mg/mL. Specifically, non-limiting examples include 50.1 mg/mL, 50.2 mg/mL, 50.3 mg/mL, 50.4 mg/mL, 50.5 mg/mL, 50.6 mg/mL, 50.7 mg/mL, 50.8 mg/mL, 50.81 mg/mL, 50.82 mg/mL, 50.83 mg/mL, 50.84 mg/mL, 50.85 mg/mL, 50.86 mg/mL, 50.87 mg/mL, 50.88 mg/mL, 50.89 mg/mL, 50.9 mg/mL, 50.9 mg/mL, 50.91 mg/mL, 50.92 mg/mL, 50.93 mg/mL, 50.94 mg/mL, 50.95 mg/mL, 50.96 mg/mL, 50.97 mg/mL, 50.98 mg/mL, 50.99 mg/mL, 51 mg/mL and any range between these point values. The protein concentration is the concentration of the antibody fraction in the antibody-drug conjugate.

In optional embodiments, the concentration of buffer in the pharmaceutical composition is about 15 mM to about 50 mM, with non-limiting examples including 15 mM, 16 mM, 17 mM, 18 mM, 19 mM, 20 mM, 30 mM, 40 mM, 50 mM and any range between these point values, preferably about 15 mM to about 50 mM, more preferably about 20 mM to about 40 mM, and most preferably about 30 mM.

In optional embodiments, the drug loading capacity (drug-to-antibody ratio; DAR) range may be the average number of cytotoxic drugs bound to each antibody h1702DS. In non-limiting examples, such average number is about 1, 2, 3, 4, 5, 6, 7, 8, 9, 10, 11, 12 or any range between these point values. The number of bound cytotoxic drugs is preferably about 3 to 8, about 4 to 8, about 5 to 7 and about 4 to 6, more preferably about 3 to 5, and most preferably 4.1.n is a decimal or integer.

In optional embodiments, the drug loading capacity (DAR) is about 4.

In optional embodiments, the pharmaceutical composition comprises:

(a) the antibody-drug conjugate at about 10 mg/mL to about 70 mg/mL, (b) polysorbate at about 0.1 mg/mL to about 0.8 mg/mL, (c) sugar at about 25 mg/mL to about 80 mg/mL, (d) glycine at about 5 mg/mL - 10 mg/mL and (e) succinate buffer at about 15 mM to about 50 mM; the pH of the composition is about 5.0 - 6.0;

Preferably, the pharmaceutical composition comprises the following components:

(a) the antibody-drug conjugate at about 20 mg/mL to about 70 mg/mL, (b) polysorbate at about 0.2 mg/mL to about 0.8 mg/mL, (c) sugar at about 25 mg/mL to about 80 mg/mL, (d) glycine at about 7 mg/mL - 8 mg/mL and (e) succinate buffer at about 10 mM to about 40 mM; the pH of the composition is about 5.0 - 6.0;

More preferably, the pharmaceutical composition comprises the following components:

(a) the antibody-drug conjugate at about 20 mg/mL to about 50 mg/mL, (b) polysorbate 80 at about 0.2 mg/mL to about 0.8 mg/mL, (c) sucrose at about 40 mg/mL to about 80 mg/mL, (d) glycine at about 7 mg/mL to about 8 mg/mL and (e) succinic acid-sodium succinate at about 20 mM to about 40 mM; the pH of the composition is about 5.0 - 6.0;

Further preferably, the pharmaceutical composition comprises the following components:

(a) the antibody-drug conjugate at about 20 mg/mL to about 30 mg/mL, (b) polysorbate 80 at about 0.2 mg/mL, (c) sucrose at about 40 mg/mL, (d) glycine at about 7.6 mg/mL and (e) succinic acid-sodium succinate at about 30 mM; the pH of the composition is about 5.5 - 5.6;

(a) the antibody-drug conjugate at about 30 mg/mL to about 50 mg/mL, (b) polysorbate 80 at about 0.2 mg/mL, (c) sucrose at about 40 mg/mL, (d) glycine at about 7.6 mg/mL and (e) succinic acid-sodium succinate at about 30 mM; the pH of the composition is about 5.5 - 5.6;

Even further preferably, the pharmaceutical composition comprises the following components:

(a) the antibody-drug conjugate at about 20 mg/mL, (b) polysorbate 80 at about 0.2 mg/mL, (c) sucrose at about 40 mg/mL, (d) glycine at about 7.6 mg/mL and (e) succinic acid-sodium succinate at about 30 mM; the pH of the composition is about 5.5 - 5.6.
(a) the antibody-drug conjugate at about 30 mg/mL, (b) polysorbate 80 at about 0.2 mg/mL, (c) sucrose at about 40 mg/mL, (d) glycine at about 7.6 mg/mL, and (e) succinic acid-sodium succinate at about 30 mM; the pH of the composition is about 5.5 - 5.6.
(a) the antibody-drug conjugate at about 50 mg/mL, (b) polysorbate 80 at about 0.2 mg/mL, (c) sucrose at about 40 mg/mL, (d) glycine at about 7.6 mg/mL, and (e) succinic acid-sodium succinate at about 30 mM; the pH of the composition is about 5.5 - 5.6.

In optional embodiments, the pharmaceutical composition described in any of the foregoing is liquid formulation.
The present disclosure also relates to a lyophilised formulation containing the antibody-drug conjugate, which is characterised in that it can form the pharmaceutical composition described above upon reconstitution.
This disclosure also relates to a method to prepare the lyophilised formulation containing the antibody-drug conjugate, which includes the lyophilisation step of the pharmaceutical composition described above.
In optional embodiments, the lyophilisation step in the method to prepare the lyophilised formulation containing the antibody-drug conjugate includes pre-freezing, primary drying and secondary drying steps in sequence. The lyophilisation is performed by freezing the formulation and subsequently sublimating water at a temperature suitable for primary drying. Under this condition, the product temperature is below the low eutectic point or collapse temperature of the formulation. Typically, the temperature range for primary drying is about -30°C to 25°C (assuming the product remains frozen during primary drying). The formulation, the size and type of the container containing sample (such as glass vials), and the volume of liquid determine the time required for drying, which can range from several hours to several days (such as 40 - 60 hours). The secondary drying phase can occur at about 0 - 40°C, mainly depending on the type and size of the container and the type of protein used. The duration of secondary drying is determined by the desired residual moisture level in the product, and is typically at least about five hours. Generally, the moisture content of formulations lyophilised under low pressure is less than about 5%, preferably less than about 3%. The pressure may be the same as that applied during the primary drying step, but preferably, the pressure for secondary drying is lower than that for primary drying. The lyophilisation conditions can vary with the formulation and vial size.
In an optional example of the present disclosure, 5 mL drug substance of the composition is lyophilised, with the lyophilisation procedure as follows: The pre-freezing temperatures are 5°C and -45°C in sequence; the temperature is -20°C and the vacuum degree is 10 Pa for primary drying; the temperature is 25°C and the vacuum degrees are 10 Pa and 1 Pa in sequence for secondary drying.
In some embodiments, the lyophilised formulation is stable at 2 - 8°C for at least 16 days, at least one month, at least three months, at least six months, at least 12 months, at least 18 months or at least 24 months. In some embodiments, the lyophilised formulation is stable at 40°C for at least seven days, at least 14 days, at least 28 days or at least 30 days.
The present disclosure also relates to a lyophilised formulation containing the antibody-drug conjugate, which is obtained through lyophilisation of the above-mentioned pharmaceutical composition comprising the antibody-drug conjugate.
The present disclosure further relates to a reconstituted solution containing the antibody-drug conjugate, which is characterised in that it is obtained by reconstituting the above-mentioned lyophilised formulation.

In optional embodiments, the reconstituted solution comprises:
(a) the antibody-drug conjugate at about 10 mg/mL to about 70 mg/mL, (b) polysorbate at about 0.1 mg/mL to about 0.8 mg/mL, (c) sugar at about 25 mg/mL to about 80 mg/mL, (d) glycine at about 5 mg/mL - 10 mg/mL, and (e) succinate buffer at about 15 mM to about 50 mM; the pH of the reconstituted solution is about 5.0 - 6.0;
Preferably, the reconstituted solution comprises the following components:
(a) the antibody-drug conjugate at about 20 mg/mL to about 70 mg/mL, (b) polysorbate at about 0.2 mg/mL to about 0.8 mg/mL, (c) sugar at about 25 mg/mL to about 80 mg/mL, (d) glycine at about 7 mg/mL to about 8 mg/mL, and (e) succinate buffer at about 10 mM to about 40 mM; the pH of the composition is about 5.0 - 6.0;
More preferably, the reconstituted solution comprises the following components:
(a) the antibody-drug conjugate at about 20 mg/mL to about 50 mg/mL, (b) polysorbate 80 at about 0.2 mg/mL to about 0.8 mg/mL, (c) sucrose at about 40 mg/mL to about 60 mg/mL, (d) glycine at about 7 mg/mL to about 8 mg/mL and (e)

succinic acid-sodium succinate at about 20 mM to about 40 mM; the pH of the reconstituted solution is about 5.0 - 6.0; Further preferably, the reconstituted solution comprises the following components:

(a) the antibody-drug conjugate at about 20 mg/mL to about 30 mg/mL, (b) polysorbate 80 at about 0.2 mg/mL, (c) sucrose at about 40 mg/mL, (d) glycine at about 7.6 mg/mL and (e) succinic acid-sodium succinate at about 30 mM; the pH of the composition is about 5.5 - 5.6; or
(a) the antibody-drug conjugate at about 30 mg/mL to about 50 mg/mL, (b) polysorbate 80 at about 0.2 mg/mL, (c) sucrose at about 40 mg/mL, (d) glycine at about 7.6 mg/mL and (e) succinic acid-sodium succinate at about 30 mM; the pH of the composition is about 5.5 - 5.6.

Even further preferably, the reconstituted solution comprises the following components:

(a) the antibody-drug conjugate at about 20 mg/mL, (b) polysorbate 80 at about 0.2 mg/mL, (c) sucrose at about 40 mg/mL, (d) glycine at about 7.6 mg/mL and (e) succinic acid-sodium succinate at about 30 mM; the pH of the composition is about 5.5 - 5.6;
(a) the antibody-drug conjugate at about 30 mg/mL, (b) polysorbate 80 at about 0.2 mg/mL, (c) sucrose at about 40 mg/mL, (d) glycine at about 7.6 mg/mL and (e) succinic acid-sodium succinate at about 30 mM; the pH of the composition is about 5.5 - 5.6;
(a) the antibody-drug conjugate at about 50 mg/mL, (b) polysorbate 80 at about 0.2 mg/mL, (c) sucrose at about 40 mg/mL, (d) glycine at about 7.6 mg/mL and (e) succinic acid-sodium succinate at about 30 mM; the pH of the composition is about 5.5 - 5.6.

The present disclosure also relates to a method to prepare the above reconstituted solution, which includes the step to reconstitute the aforementioned lyophilised formulation, and the solution used for reconstitution includes but is not limited to water for injection, saline or glucose solution.
The present disclosure further relates to a product comprising a container, which contains the aforementioned pharmaceutical composition, lyophilised formulation or reconstituted solution. In some embodiments, the container is a neutral borosilicate glass vial for injection.
The present disclosure also relates to the use of the aforementioned pharmaceutical composition or lyophilised formulation or reconstituted solution or product in the preparation of medicament for the treatment or prevention of tumours.
The present disclosure also relates to a method to treat diseases, including provision of the aforementioned pharmaceutical composition or lyophilised formulation or reconstituted solution or product.
The present disclosure also relates to the aforementioned pharmaceutical composition, or lyophilised formulation, or reconstituted solution, or product as medicament, preferably for the treatment or prevention of tumour diseases.
In optional embodiments, the diseases or tumours are cancers associated with the expression of HER2, HER3, B7H3 or EGFR.
In optional embodiments, the cancer is breast cancer, ovarian cancer, cervical cancer, uterine cancer, prostate cancer, kidney cancer, urethral cancer, bladder cancer, liver cancer, gastric cancer, endometrial cancer, salivary gland cancer, esophagoeal cancer, melanoma, glioma, neuroblastoma, sarcoma, lung cancer, colon cancer, rectal cancer, colorectal cancer, leukaemia, bone cancer, skin cancer, thyroid cancer, pancreatic cancer or lymphoma.
As the person skilled in the art will appreciate, one, several or all properties of various embodiments described in the present disclosure may be further combined to form other embodiments to the present disclosure. The above embodiments to the present disclosure and other embodiments obtained by combination are further illustrated by the detailed description below.
For the development of ADC formulation, even under low concentration conditions, inter-chain disulfide bonds opened during conjugation may oxidise, and the strongly hydrophobic toxin conjugated also affects the stability of the antibody to some extent. If the concentration is increased, this effect will inevitably be intensified, causing the ADC more prone to aggregation/degradation and unable to keep stable in low concentration formulations. We have screened suitable high-concentration formulae through rational formula design, thus maintaining the stability of ADC. Furthermore, the high-concentration ADC formulation of the present invention may have a wider safe therapeutic window in clinical practice while greatly shortening production time, reducing costs of consumables (vials, stoppers, aluminum caps, etc.), and improving the convenience of clinical administration for healthcare professionals.

## Detailed Description of Invention

The present disclosure involves a pharmaceutical composition with stable performance that is more conducive to production and administration. Wherein, the undesirable instability may include any one or more of the following:

aggregation, deamidation (such as Asn deamidation), oxidation (such as Met oxidation), isomerisation (such as Asp isomerisation), clipping/hydrolysis/fragmentation (such as hinge region fragmentation), succinimide formation, unpaired cysteine and dissociation of toxins. Specifically, the pharmaceutical composition described in the present disclosure contains the antibody-drug conjugate and the buffer.

**Terms**

To make it easier to understand the present disclosure, certain technical and scientific terms are specifically defined below. Unless otherwise clearly defined herein, all other technical and scientific terms used herein have the meanings generally understandable to the ordinary technician skilled in the art of the present disclosure.

The present disclosure incorporates by reference the entire content of WO2020063673 into the present application.

An antibody-drug conjugate (ADC) is a compound that link antibodies to bioactive cytotoxins or small molecule drugs with cell-killing activity through linker units.

The "drug loading capacity" is also known as the drug-to-antibody ratio (DAR), i.e., the average number of drugs conjugated to each antibody in ADC. The number of drugs conjugated to each antibody can range from about 1 to about 10, and in some examples, from about 1 to about 8, preferably in the range of 2 - 8, 2 - 7, 2 - 6, 2 - 5, 2 - 4, 3 - 4, 3 - 5, 5 - 6, 5 - 7, 5 - 8 and 6 - 8.For example, the drug loading capacity can be an average of 1, 2, 3, 4, 5, 6, 7, 8, 9 and 10.The generic formula of ADC in the present disclosure includes a collection of antibodies conjugated to drugs within the aforementioned certain ranges. In the embodiments in the present disclosure, the drug loading capacity can be expressed as n. The drug loading capacity can be determined using conventional methods such as ultraviolet (UV)/visible spectrometry, mass spectrometry, enzyme-linked immunosorbent assay (ELISA) and high-performance liquid chromatography (HPLC).

The term "linker unit" or "linking fragment" or "linking unit" refers to a chemical structure fragment or bond that is linked toantibody or its antigen-binding fragment at one end and to drug at the other end, and may also connect to other linkers before being connected to drug.

Linkers, including extensions, spacers and amino acid units, can be synthesised using methods known in the art, such as those described in US2005-0238649A1. Linkers can be "cleavable linkers" that facilitate drug release within cells. For example, acid-labile linkers (such as hydrazone), protease-sensitive (such as peptidase-sensitive) linkers, photolabile linkers, dimethyl linkers or disulfide-containing linkers (Chari et al., Cancer Research 52: 127-131 (1992); U.S. Patent No. 5,208,020) can be used.

The following non-limiting methods can be used to control the loading capacity of cytotoxic drugs, including:

(1) controlling the molar ratio of linking agents to monoclonal antibodies,
(2) controlling reaction time and temperature,
(3) selecting different reaction reagents.

The three-letter codes and one-letter codes of amino acids used in the present disclosure are as described in J. biol. chem, 243, p3558 (1968).

The "antibody" described in the present disclosure is used in the broadest sense and encompasses various antibody structures, including but not limited to full-length antibodies and antibody fragments (or antigen-binding fragments or antigen-binding fraction), as long as they exhibit the desired antigen-binding activity. Typically, a natural complete antibody consists of two identical heavy chains and two identical light chains connected by inter-chain disulfide bonds, forming a four-peptide chain structure.

The engineered antibodies or antigen-binding fragments in the present disclosure can be prepared and purified using conventional methods. For example, cDNA sequences encoding the heavy and light chains can be cloned and recombined into GS expression vectors. The recombinant immunoglobulin expression vectors can be stably transfected into CHO cells. As a more preferred prior art, mammalian expression systems result in glycosylation of antibodies, particularly at the highly conserved N-terminal sites in the Fc region. Positive clones are expanded in serum-free media in bioreactors to produce antibodies. The culture medium containing the secreted antibodies can be purified using conventional techniques. For example, the purification can be performed using A or G Sepharose FF columns containing adjusted buffers. Non-specifically bound components are washed away. The bound antibodies can then be eluted using the pH gradient method, with antibody fragments detected by sodium dodecyl sulfate polyacrylamide gel electrophoresis (SDS-PAGE) and collected. Antibodies can be filtered and concentrated using conventional methods. Soluble mixtures and polymers can also be removed using conventional methods such as molecular sieve and ion exchange. The resulting product should be immediately frozen, for example, at -70°C, or lyophilised.

"Buffer" refers to a buffer that can withstand pH changes due to the effect of acidbase conjugate components. The examples of buffers that control pH within an appropriate range include acetate, succinate, gluconate, histidine, oxalate, lactate, phosphate, citrate, tartrate, fumarate, glycylglycine and other organic acid buffers.

"Histidine buffer" is a buffer that contains histidine ions. The examples of histidine buffer include histidine-hydrochloride, histidine-acetate, histidine-phosphate and histidine-sulfate, preferably histidine-acetate buffer, which is prepared from histidine and acetic acid, while histidine hydrochloride buffer is prepared from histidine and hydrochloric acid.

"Citrate buffer" is a buffer that contains citrate ions. The examples of citrate buffer include citric acid-sodium citrate, citric acid-potassium citrate, citric acid-calcium citrate and citric acid-magnesium citrate. The preferred citrate buffer is citric acid-sodium citrate.

"Succinate buffer" is a buffer that contains succinate ions. The examples of succinate buffer include succinic acid-sodium succinate, succinic acid-potassium succinate and succinic acid-calcium succinate. The preferred succinate buffer is succinic acid-sodium succinate. For example, the succinic acid-sodium succinate can be prepared from succinic acid and sodium hydroxide, or from succinic acid and sodium succinate.

"Phosphate buffer" is a buffer that contains phosphate ions. The examples of phosphate buffer include disodium hydrogen phosphate-sodium dihydrogen phosphate, disodium hydrogen phosphate-potassium dihydrogen phosphate and disodium hydrogen phosphate-citric acid. The preferred phosphate buffer is disodium hydrogen phosphate-sodium dihydrogen phosphate.

"Acetate buffer" is a buffer that contains acetate ions. The examples of acetate buffer include acetic acid-sodium acetate, histidine acetate, acetic acid-potassium acetate, acetic acid-calcium acetate and acetic acid-magnesium acetate. The preferred acetate buffer is acetic acid-sodium acetate.

"Pharmaceutical composition" refers to a mixture containing one or more antibody-drug conjugate(s) described herein or their physiologically/pharmaceutically acceptable salts or prodrugs along with other chemical components, such as physiologically/pharmaceutically acceptable carriers and excipients. The purpose of pharmaceutical composition is to maintain the stability of active antibody component, facilitate the administration to biological organism, and promote the absorption of active component to exert their biological activity.

In the present disclosure, "pharmaceutical composition" and "formulation" are not mutually exclusive.

The solution form of the pharmaceutical composition described in the present disclosure contains water as solvent unless otherwise specified.

"Lyophilised formulation" refers to the formulation or pharmaceutical composition obtained after vacuum lyophilisation steps from a liquid or solution form of the pharmaceutical composition or liquid or solution formulation.

The term "about" or "approximately" used herein refers that values are within acceptable margins of error of specific values determined by the general persons skilled in the art, and the numerical part may depend on how it is measured or determined (i.e., the limits of the measurement system).For instance, "about" in each embodiment in this field may mean that the standard deviation is within 1 or more than 1. Alternatively, "about" or "substantially contain" may mean up to a 20% range. Moreover, particularly for biological systems or processes, this term can mean up to an order of magnitude or up to five times a value. Unless otherwise specified, when a specific value appears in this application and claims, "about" or "substantially contain" should mean the specific value is assumed to be within the acceptable margin of error.

The pharmaceutical composition described in the present disclosure can achieve a stable effect: For the pharmaceutical composition of which the antibody-drug conjugate substantially retains its physical stability and/or chemical stability and/or biological activity after storage, preferably, the pharmaceutical composition substantially retains its physical and chemical stability as well as biological activity after storage. The storage period is generally selected based on the predetermined shelf life of the pharmaceutical composition. Currently, there are various analytical techniques available to determine protein stability, which can determine the stability after storage at selected temperatures over specified time periods.

A stable formulation is a formulation in which no significant changes are observed under the following conditions: kept at refrigerated temperature (2 - 8°C) for at least three months, preferably six months, more preferably one year, and even more preferably up to two years. Additionally, a stable liquid formulation is a liquid formulation thatexhibits desired characteristics after storage at temperatures including 25°C for time periods including one month, three months and six months. Typical examples of stability: as measured by size exclusion chromatography-high-performance liquid chromatography (SEC-HPLC), usually no more than about 10%, preferably no more than about 5% of the antibody monomers aggregate or degrade. By visual analysis, the formulation is a faint yellow to nearly colourless, clear liquid, or is colourless or clear to slightly opalescent. The concentration, pH and osmolality of the formulation exhibit no more than ±10% variation. A reduction of no more than about 10%, preferably no more than about 5%, is typically observed. Aggregation generally forms at no more than about 10%, preferably no more than about 5%.

If the antibody-drug conjugate shows no significant increase in aggregation, precipitation and/or denaturation after visual inspection of colour and/or clarity, or as measured by UV light scattering, size exclusion chromatography (SEC) and dynamic light scattering (DLS), then the antibody-drug conjugate "retains its physical stability" in the pharmaceutical formulation. The changes in protein conformation can be evaluated by fluorescence spectroscopy (which determines tertiary structure of protein) and Fourier Transform Infrared (FTIR) spectroscopy (which determines secondary structure of

protein).

If the antibody-drug conjugate shows no significant chemical changes, then the antibody "retains its chemical stability" in the pharmaceutical formulation. The chemical stability can be assessed by detecting and quantifying the protein with its forms changed chemically. The degradation processes that frequently alter the chemical structure of proteins include hydrolysis or truncation (evaluated by methods such as size exclusion chromatography and capillary electrophoresis sodium dodecyl sulfate [CE-SDS]), oxidation (evaluated by methods such as peptide mapping combined with mass spectrometry or Matrix-Assisted Laser Desorption/Ionization-Time of Flight Mass Spectrometry [MALDI/-TOF/MS]), deamidation (evaluated by methods such as ion exchange chromatography, capillary isoelectric focusing, peptide mapping and isoaspartate measurement) and isomerisation (evaluated by measuring isoaspartate content and peptide mapping).

If the biological activity of the antibody-drug conjugate at a given time is within the predetermined range of biological activity exhibited during the preparation of the pharmaceutical formulation, then the antibody-drug conjugate "retains its biological activity" in the pharmaceutical formulation.

"Optional" or "optionally" means that the event or circumstance described subsequently may or may not occur, and the description includes situations where the event or circumstance occurs or does not occur. For example, "optionally including 1 - 3 variable regions of the antibody heavy chain" means that the variable regions of the antibody heavy chain with specific sequences may or may not be present.

"Substitute" refers that one or more hydrogen atoms, preferably up to 5, and more preferably 1 - 3, in a group is (are) independently replaced by the corresponding number of substituents. It is self-evident that substituents are only in their possible chemical positions, and the persons skilled in the art can determine (either experimentally or theoretically) which substitutions are possible or impossible without excessive effort. For example, the binding of amino group or hydroxyl group with free hydrogen to a carbon atom with an unsaturated (such as olefinic) bond may be unstable.

The preparation of conventional pharmaceutical compositions is described in the Chinese Pharmacopoeia.

The term "carrier" used for the drugs in the present disclosure refers to a system that can change the way a drug enters human body and its distribution in the body, control the release rate of the drug, and deliver the drug to target organs. The drug carrier release and targeting system can decrease drug degradation and loss, reduce side effects and improve bioavailability. For example, high molecular surfactants that can serve as carriers can selfassemble due to their unique amphiphilic structure, forming various types of aggregates, preferably micelles, microemulsions, gels, liquid crystals and vesicles. These aggregates can encapsulate drug molecules while also exhibiting good permeability to membranes, making them good drug carriers.

"Administration" and "treatment", when applied to animals, humans, experimental subjects, cells, tissues, organs or biological fluids, refer to the contact of exogenous drugs, therapeutic agents, diagnostic agents or compositions with animals, humans, subjects, cells, tissues, organs or biological fluids."Administration" and "treatment" can refer to, for example, therapy, pharmacokinetics, diagnosis, research and experimental methods. The treatment of cells includes the contact between reagents and cells, and between reagents and fluids, where the fluids are in contact with cells."Administration" and "treatment" also imply treatment through reagents, diagnostics, conjugated compositions or another form of in vitro and ex vivo treatment, such as cells." Treatment", when applied to humans, veterinary or research subjects, refers to therapeutic treatments, preventive or prophylactic measures, research and diagnostic applications.

"Treatment" means administration of therapeutic agents for internal or topical use to a patient, such as a composition containing any of the conjugated compounds in the present disclosure, where the patient has one or more disease symptoms and the therapeutic agents are known to have a therapeutic effect on those symptoms. Generally, a therapeutic agent is administered in an amount effective to alleviate one or more disease symptoms in the treated patient or population to induce regression of such symptoms or to inhibit their progression to any clinically measurable degree. The amount of the therapeutic agent that effectively alleviates any specific disease symptom (also referred to as "therapeutically effective amount") may vary depending on several factors, such as the patient's disease state, age and body weight, as well as the drug's ability to produce the desired effect to the patient. Whether the disease symptom has alleviated can be evaluated by any clinical testing method commonly used by physicians or other healthcare professionals to evaluate the severity or progression of the symptom. Although the embodiments (such as therapeutic methods or products) in the present disclosure may be ineffective in alleviating each targeted disease symptom, yet any statistical testing method known in the field, such as Student's t-test, chi-square test, Mann-Whitney U test, Kruskal-Wallis test (H-test), Jonckheere-Terpstra test and Wilcoxon test, can determine that they should alleviate the targeted disease symptoms in a statistically significant number of patients.

An "effective amount" refers to an amount sufficient to improve or prevent the symptoms or conditions of a medical disorder. An effective amount also refers to an amount sufficient to allow for or facilitate diagnosis. The effective amount for a specific patient or veterinary subject may vary depending on the following factors: such as the condition to be treated, the general health of the patient, the route and dosage of administration, and the severity of side effects. The effective amount may be the maximum dosage or dosing regimen that avoids significant side effects or toxic effects.

"Replacement" refers to the replacement of the solvent system dissolving antibody proteins, such as using the

buffer system of a stable formulation to replace a high-salt or hyperosmotic solvent system containing antibody proteins through physical operations, thus allowing for the presence of antibody proteins in the stable formulation. The socalled physical operations include but are not limited to ultrafiltration, dialysis, or reconstitution after centrifugation.

## Specific Embodiments

The following further describes the present disclosure in combination with examples, but these examples are not intended to limit the scope of the present disclosure. The experimental methods without specific conditions in the examples of the present disclosure are generally carried out under conventional conditions, such as those referenced in the Cold Spring Harbor Laboratory's "Antibody Experimental Manual" and "Molecular Cloning Manual"; or the conditions recommended by the raw material or product manufacturers. The reagents of which the specific source is not indicated are commercially available conventional reagents.

## Biological Evaluation

I. Preparation of anti-B7H3-ADC formulation ADC-1

The structure of anti-B7H3-ADC in the present disclosure is as follows:

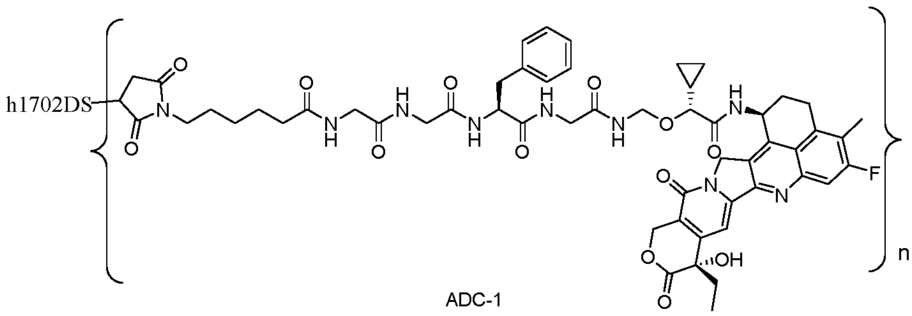

ADC-1

Firstly, dilute the naked antibody solution h1702DS (6.43 L, 1.39 mmol) with pH 5.0 acetic acid-sodium acetate buffer (0.98 L, 30 mM), then sequentially add ethylene diamine tetraacetic acid (EDTA) solution (0.57 L, 29 mmol), pH 8.2 histidineacetic acid-Tris solution (1.47 L, 20 mM) and Tris solution (0.2 L, 1M), mix well, then add tris (2-carboxyethyl) phosphine (TCEP) solution (1.78 L, 3.06 mmol), mix well, and let the resulting solution react at 12°C for two hours; add acetic acid to adjust the pH to 6.0 after the end of reaction.

Firstly, add 0.57 L dimethyl sulfoxide (DMSO) solvent to the above reaction solution, then add the compound 5-A solution (prepared by reference to 5-A in Example 5 of WO2020063673A1 Description, where the compound 5-A is dissolved in DMSO, 6.58 g, 6.12 mmol) dropwise to the reaction solution, mix well, and let the resulting solution react at 12°C for one hour. After the end of reaction, add acetic acid to adjust the pH of the reaction solution to 5.0.The reaction solution was purified by cation chromatography (column resin Capto S impAct, elution phase 50 mM acetic acid-sodium acetate + 240 mM NaCl, pH 5.5), and then underwent ultrafiltration exchange, resulting in the exemplary product ADC-1 of B7H3-ADC antibody-drug conjugate in the present disclosure.

The DAR value was detected by RP-HPLC, with n being 4.1.

## II. Formulation

**The equipment used in the preparation and detection of formulation and the methods for calculation of results are as follows:**

### 1) Size Exclusion Chromatography (SEC):

A method for analysis of the separation of solutes based on the relative relationship between the pore size of the gel pore and the molecule coil size of the high molecule samples.

$$\text{SEC\% (percentage of SEC monomer content)} = \text{A monomer/A total x 100\%}$$

(A monomer is the peak area of the main peak monomer in the sample, A total is the sum of the areas of all peaks.))

SEC measuring instrument: Agilent 1260; Column: Waters, XBridge BEH200Å SEC (300 × 7.8 mm, 3.5 μm)

**2) Capillary Electrophoresis (CE):**

A type of electrophoresis where the gel is transferred into a capillary as a supporting medium, and also a method of separation based on the sample molecular weight under a certain voltage.

Percentage of reduced CE purity = A main peak/A total x 100% (A main peak is the peak area of the light chain main peak + heavy chain main peak in the sample, A total is the sum of the areas of all peaks.)

CE measuring instrument: Beckman model Plus800

**3) Turbidity determination:**

The extent to which light is obstructed as it passes through water layer, indicating the ability of the water layer to scatter and absorb light, which relates not only to the content of suspended solids, but also to the particle composition, size, shape and surface reflectivity. By comparing the absorbance values of the same protein sample at the same concentration and wavelength (near UV and visible wavelength range), a higher absorbance value indicates greater turbidity and a more pronounced tendency of protein aggregation in the sample. The measuring instrument is a multifunctional microplate reader (Molecular Devices M5), to which the same volume of sample was added to a 96-well plate to read the absorbance value.

**4) Osmotic pressure determination:**

Osmotic pressure was determined using the freezing point method. Based on the directly proportional relationship between the freezing point depression and the molar concentration of the solution, the highly sensitive temperature-sensitive components were used to determine the freezing point of the solution and convert the electrical quantity into osmotic pressure. Instrument manufacturer: Loser, model OM815.

**5) Protein concentration determination:**

The concentration of the antibody-drug conjugate in the present disclosure was measured by protein concentration, i.e., the concentration of the antibody fraction in the antibody-drug conjugate.

As the toxin in the antibody-drug conjugate is absorbed at the characteristic protein absorption wavelength of 280 nm, and also at 370 nm, the following formula is used to calculate the aforementioned protein concentration:

$$A_{280nm} = \left(C_{drug} \times E_{drug-280} + C_{mAb} \times E_{mAb-280}\right) \times l$$

$$A_{370nm} = C_{drug} \times E_{drug-370} \times l$$

Take

$$R = \frac{E_{drug-370}}{E_{drug-280}}$$

Namely:

$$C_{mAb} = \frac{R \times A_{280nm} - A_{370nm}}{R \times E_{mAb-280}}$$

Where, $A_{280nm}$: average absorbance value of a single sample of test article solution at wavelength of 280 nm when the path length is 1 cm;

$A_{370nm}$: average absorbance value of a single sample of test article solution at wavelength of 370 nm when the path length is 1 cm;

$E_{mAb-280}$: mass extinction coefficient of protein at wavelength of 280 nm, which is 1.532 $g^{-1}cm^{-1}L$;

$E_{drug-280}$: mass extinction coefficient of toxin at wavelength of 280 nm, which is 5.17 $g^{-1}cm^{-1}L$;

$E_{drug-370}$: mass extinction coefficient of toxin at wavelength of 370 nm, which is 17.89 $g^{-1}cm^{-1}L$;

R: ratio of extinction coefficients of toxin at 370 nm to 280 nm, which is 3.46;
$C_{mAb}$: protein concentration, mg/mL;
l: Path length, cm (in this case, the path length is 1 cm).

If the test solution is diluted, then the protein concentration is: C (mg/mL) = $C_{mAb} \times N$, N is the dilution factor.
Instrument for determination of protein concentration: UV-visible spectrophotometer, model: Nano Drop One.

**6) Calculation of drug loading capacity (reversed phase high-performance liquid chromatography, RP-HPLC)**

**1. Reagents and instruments:**

HPLC system: Waters H-Class ultra-high performance liquid chromatography (UPLC) system
Detector: TUV detector (measuring wavelength:280 nm)
Chromatography column: BioResolve RP mAb Polyphenyl (2.7μm 4.6*150 mm)

**2. Detection conditions:**

Column temperature: 80°C
Flow rate: 1.0 mL/minute
Mobile phaseA: 0.1% formic acid + 0.025% trifluoroacetic acid (TFA) in aqueous solution.
Mobile phaseB: 0.1% FA + 0.025% trifluoroacetic acid (TFA) in acetonitrile solution.
Gradient procedure: 27.0% B - 45.0% B (0.00 minute - 12.00 minutes), 45.0% B - 80.0% B (12.00 minutes - 13.00 minutes), 80.0% B - 80.0% B (13.00 minutes - 15.00 minutes), 27.0% B - 27.0% B (15.04 minutes - 20.00 minutes),
Injection volume: 5.0μL

**3. Data analysis**

Compared with the antibody light chain ($L_0$) and antibody heavy chain ($H_0$) not bound to drug, the light chain bound to drug (light chain bound to one drug: $L_1$) and the heavy chain bound to drugs (heavy chain bound to one drug: $H_1$, heavy chain bound to two drugs: $H_2$, heavy chain bound to three drugs: $H_3$, and heavy chain bound to four drugs: $H_4$) exhibited a directly proportional increase in hydrophobicity with the number of bound drugs, and a longer retention time. Therefore, elution can be performed in an order of $L_0$, $L_1$, $H_0$, $H_1$, $H_2$, $H_3$ and $H_4$. As a comparison, the results of the retention time between $L_0$ and $H_0$ were based to assign the detected peaks to any of $L_0$, $L_1$, $H_0$, $H_1$, $H_2$, $H_3$ and $H_4$.
Due to the absorption of UV by drug linker, the molar absorption coefficients of the light chain, heavy chain and drug linker were used to correct the obtained peak areas depending on the number of bound drugs, based on the following expressions. The calculation formulas are as follows:

Light chain (εLC-280)/(εLC-280 + number of bound drugs x εdrug-280)
Heavy chain (εHC-280)/(εHC-280 + number of bound drugs x εdrug-280)
Notes: εLC-280: Molar extinction coefficient of light chain at 280 nm;
εHC-28: Molar extinction coefficient of heavy chain at 280 nm;
εdrug-280: Molar extinction coefficient of toxin at 280 nm.

Table 1. Table for Calculation of Drug Loading Capacity by Reversed Phase Chromatography

| Name | Number of Bound Drugs | Percentage of Corrected Peak Area |
|---|---|---|
| $L_0$ | 0 | 100 x corrected peak area of $L_0$/sum of corrected peak areas of LC |
| $L_1$ | 1 | 100 x corrected peak area of $L_1$/sum of corrected peak areas of LC |
| $H_0$ | 0 | 100 x corrected peak area of $H_0$/sum of corrected peak areas of HC |
| $H_1$ | 1 | 100 x corrected peak area of $H_1$/sum of corrected peak areas of HC |
| $H_2$ | 2 | 100 x corrected peak area of $H_2$/sum of corrected peak areas of HC |

(continued)

| Name | Number of Bound Drugs | Percentage of Corrected Peak Area |
|---|---|---|
| $H_3$ | 3 | 100 x corrected peak area of $H_3$/sum of corrected peak areas of HC |
| $H_4$ | 4 | 100 x corrected peak area of $H_4$/sum of corrected peak areas of HC |
| Notes: Sum of corrected peak areas of LC = corrected peak area of $L_0$ + corrected peak area of $L_1$ | | |

Sum of corrected peak areas of HC = corrected peak area of $H_0$ + corrected peak area of $H_1$ + corrected peak area of $H_2$ + corrected peak area of $H_3$ + corrected peak area of $H_4$, the drug loading capacity of this ADC is calculated as follows:

$$\text{Drug loading capacity n} = 2 \times \sum [(\text{number of bound drugs} \times \text{percentage of corrected peak area})/100]$$

**Example 1: Anti-B7H3-ADC Antibody Formulation Buffer System and pH Screening**

The following buffers were used to prepare an anti-B7H3-ADC formulation containing 60 mg/mL sucrose, 0.2 mg/mL polysorbate 80 (abbreviated as: PS80) and ADC-1 at a protein concentration of 20 mg/mL, where the buffers are as follows:

1) 10 mM citric acid-sodium citrate (abbreviated as: CA), pH 5.0;
2) 10 mM succinic acid-sodium succinate (abbreviated as: SA), pH 5.0;
3) 10 mM SA, pH 5.5;
4) 10 mM histidine-hydrochloride (abbreviated as: His), pH 5.5;
5) 10 mM His, pH 6.0;
6) 10 mM His, pH 6.5;
7) 10 mM histidine-acetate (abbreviated as: His-AA), pH 5.0;
8) 10 mM His-AA, pH 5.5;
9) 10 mM disodium hydrogen phosphate-sodium dihydrogen phosphate (abbreviated as: PB), pH 6.5.

The prepared formulation was filtered, filled, sealed and capped. The stability of the samples kept under forced degradation conditions (40°C M1, i.e., placed at a high temperature of 40°C for one month; shaken at 300 rpm for D10, i.e., shaking for 10 days) was investigated, and the stability of formulation was investigated with the appearance, SEC and reduced CE-SDS as evaluation indicators. The experimental results are shown in Table 2.

Under Shaking D10 forced degradation conditions, the appearance of the protein formulations in the SA and His-AA buffer systems was better than that in the CA, His and PB systems; the SA, His-AA, CA and His buffer systems showed no significant difference in purity, and all were superior to the PB system; under 40°C M1 forced degradation conditions, the protein formulations in the SA and His-AA buffer systems had better appearance than in the CA and PB systems. In overall consideration, SA and His-AA were preferred as the buffer systems of B7H3-ADC formulation for subsequent formula screening, with the preferred pH value of 5.0 - 5.5, more preferably 5.5.

Table 2. Screening Experiment Results of pH and Buffer System

| Serial No. | Storage Conditions | Appearance | SEC Monomer% | Reduced CE-SDS% |
|---|---|---|---|---|
| 1 | D0 | Clear | 95.1 | 97.3 |
| | Shaking D10 | A large number of particles | 89.9 | 96.3 |
| | 40°C M1 | Opalescent | 70.8 | 90.7 |
| 2 | D0 | Clear | 95.6 | 97.3 |
| | Shaking D10 | Clear | 91.4 | 96.2 |
| | 40°C M1 | Clear | 75.1 | 87.4 |
| 3 | D0 | Clear | 95.2 | 97.2 |
| | Shaking D10 | Clear | 90.4 | 96.1 |
| | 40°C M1 | Clear | 77.7 | 91.0 |

(continued)

| Serial No. | Storage Conditions | Appearance | SEC Monomer% | Reduced CE-SDS% |
|---|---|---|---|---|
| 4 | D0 | Clear | 95.7 | 97.2 |
| | Shaking D10 | Many particles | 91.4 | 96.0 |
| | 40°C M1 | Clear | 82.6 | 90.7 |
| 5 | D0 | Clear | 95.2 | 97.2 |
| | Shaking D10 | White cloudy, full vial of particles | 91.0 | 95.8 |
| | 40°C M1 | Particles | 83.6 | 92.6 |
| 6 | D0 | Clear | 94.7 | 97.1 |
| | Shaking D10 | White cloudy, full vial of particles | 89.4 | 93.4 |
| | 40°C M1 | Cloudy | 83.9 | 92.6 |
| 7 | D0 | Clear | 96.2 | 97.3 |
| | Shaking D10 | Clear | 92.9 | 96.2 |
| | 40°C M1 | Few particles | 82.8 | 89.7 |
| 8 | D0 | Clear | 95.9 | 97.4 |
| | Shaking D10 | Clear | 91.4 | 95.9 |
| | 40°C M1 | Clear | 83.3 | 92.0 |
| 9 | D0 | Clear | 93.3 | 96.5 |
| | Shaking D10 | A large number of particles | 85.1 | 95.0 |
| | 40°C M1 | Particles | 78.1 | 90.2 |
| Notes: D0 indicates the start of the experiment; "M" indicates month, for example, M1 indicates one month | | | | |

**Example 2: Screening of Surfactant in Anti-B7H3-ADC Antibody Formulation**

The 10 mM SA buffer system at pH 5.0 was used to prepare an anti-B7H3-ADC formulation containing 60 mg/mL sucrose, ADC-1 at a protein concentration of 20 mg/mL, and various types and concentrations of surfactants. Specifically:

1) 0.2 mg/mL polysorbate 80 (abbreviated as: PS80);
2) 0.4 mg/mL PS80;
3) 0.2 mg/mL polysorbate 20 (abbreviated as: PS20).

The prepared formulation was filtered, filled, sealed and capped. The samples were kept at shaking (25°C, 300 rpm, six days), freezing-thawing (-35°C to 2 - 8°C for five cycles) and high temperature (40°C M1) conditions, and the stability of formulation was investigated with the appearance, SEC and reduced-capillary electrophoresis (R-CE) as evaluation indicators. The experimental results are shown in Table 3.

The experimental results showed no significant differences in appearance and purity among the 0.2 mg/mL PS80, 0.4 mg/mL PS80 and 0.2 mg/mL PS20 groups under the storage conditions.

Table 3. Screening Experiment Results of Surfactant in Formulation

| Serial No. | Storage Conditions | Appearance | SEC Monomer% | R-CE% |
|---|---|---|---|---|
| 1 | D0 | Clear | 97.2 | 97.9 |
| | Shaking D6 | Clear | 96.5 | 97.7 |
| | FT 5Cycle | Clear | 97.1 | 98.1 |
| | 40°C M1 | Clear | 92.4 | 94.6 |

(continued)

| Serial No. | Storage Conditions | Appearance | SEC Monomer% | R-CE% |
|---|---|---|---|---|
| 2 | D0 | Clear | 97.2 | 98.1 |
| | Shaking D6 | Clear | 96.5 | 97.2 |
| | FT 5Cycle | Clear | 96.6 | 98.3 |
| | 40°C M1 | Clear | 91.8 | 94.3 |
| 3 | D0 | Clear | 97.7 | 97.9 |
| | Shaking D6 | Clear | 96.5 | 97.6 |
| | FT 5Cycle | Clear | 96.6 | 98.0 |
| | 40°C M1 | Clear | 92.5 | 94.4 |
| Notes: In the table, "D" indicates day, for example, D6 indicates six days, D0 indicates the start of the experiment; "M" indicates month, for example, M1 indicates one month; "FT 5Cycle" indicates five freeze-thaw cycles. | | | | |

**Example 3: Screening of Sugar in Anti-B7H3-ADC Antibody Formulation**

The 10 mM SA buffer system at pH 5.0 was used to prepare an anti-B7H3-ADC formulation containing 0.2 mg/mL polysorbate 80, and ADC-1 at a protein concentration of 20 mg/mL. Different sugars are as follows:

1) 60 mg/mL sucrose;
2) 60 mg/mL trehalose;
3) 50 mg/mL mannitol.

The prepared formulation was filtered, filled, sealed and capped. The stability of the samples under forced degradation conditions (Shaking D6, i.e., shaking for six days; FT 5Cycle, i.e., five freeze-thaw cycles at -35°C to 2 - 8°C) was investigated, and the stability of the formulation was investigated with SEC as an evaluation indicator. The experimental results are shown in Table 4.

The experiment data showed that under Shaking D6 and FT5C forced degradation conditions, the SEC purity of the sucrose and trehalose groups was better than that of the mannitol group. Subsequently, the anti-B7H3-ADC antibody formulation containing 20 mg/mL antibody, 60 mg/mL sucrose and 0.2 mg/mL PS80 was prepared in 10 mM SA buffer at pH 5.0, and the formulation sample was lyophilised. After lyophilisation, the appearance showed collapse at the bottom; further optimisation was continued, with the concentration of sucrose reduced from 60 mg/mL to 40 mg/mL.

Table 4. Screening Experiment Results of Excipients

| Serial No. | Storage Conditions | Appearance | SEC Monomer% |
|---|---|---|---|
| 1 | D0 | Clear | 97.2 |
| | Shaking D6 | Clear | 96.5 |
| | FT 5Cycle | Clear | 97.1 |
| 2 | D0 | Clear | 97.2 |
| | Shaking D6 | Clear | 96.4 |
| | FT 5Cycle | Clear | 97.1 |
| 3 | D0 | Clear | 97.6 |
| | Shaking D6 | Clear | 89.5 |
| | FT 5Cycle | Clear | 83.6 |
| Notes: In the table, "D" indicates day, D0 indicates the start of the experiment. | | | |

**Example 4: Screening of Osmotic Pressure Regulator in Anti-B7H3-ADC Antibody Formulation**

The 10 mM SA buffer system at pH 5.5 was used to prepare an anti-B7H3-ADC formulation containing 40 mg/mL

sucrose, 0.2 mg/mL PS80 and ADC-1 at a protein concentration of 20 mg/mL. By increasing the ionic strength and the screening of osmotic pressure regulator, the osmotic pressure of the formulation was controlled within isotonic range (270 - 330 mosm).

The 30 mM SA buffer system at pH 5.5 was used to prepare an anti-B7H3-ADC formulation containing 40 mg/mL sucrose, 0.2 mg/mL PS80, ADC-1 at a protein concentration of 20 mg/mL, and different types of isotonic osmotic pressure regulators. Specifically:

1) 58 mM arginine hydrochloride;
2) 7.6 mg/mL glycine;

The prepared formulation was filtered, filled, partially sealed and lyophilised. The lyophilisation procedure is shown in Table 5:

Table 5. Lyophilisation Procedure

| Lyophilisation Process Parameters | Set Temperature (°C) | Set Time (Minutes) | Hold Time (Minutes) | Vacuum Degree (Pa) |
|---|---|---|---|---|
| Pre-freezing | 5 | 10 | 60 | N/A |
| Pre-freezing | -45 | 50 | 120 | N/A |
| Primary Drying | -20 | 120 | 3000 | 10 |
| Secondary Drying | 25 | 60 | 1 | 10 |
| | 25 | 1 | 600 | 1 |

The stability of the lyophilised samples under forced degradation conditions (40°C D23, i.e., placed at high temperature of 40°C for 23 days) was investigated, and the stability of the formulation was investigated with the appearance, SEC and reduced R-CE as evaluation indicators. The experimental results are shown in Table 6.

Under 40°C D23 forced degradation conditions, there were no significant differences in purity among the groups, and the appearance of the glycine group was significantly better than that of the arginine hydrochloride group. The formulation containing 40 mg/mL sucrose and 7.6 mg/mL glycine in samples had an osmotic pressure of about 300 mOsm, therefore, the preferred concentration of glycine was 7.6 mg/mL. The osmotic pressure regulator glycine was used and when its concentration was 5.3 mg/mL - 9.8 mg/mL, the osmotic pressure of formulation was controlled within the isotonic range.

Table 6. Screening Experiment Results of Osmotic Pressure Regulator in Formulation

| Serial No. | Storage Conditions | Appearance | SEC Monomer% | **R-**CE% |
|---|---|---|---|---|
| 1 | Before lyophilisation | Clear | 96.9 | 96.6 |
| | After lyophilisation | White cake, bottom collapsed | 97.2 | 95.7 |
| | 40°C D23 after lyophilisation | Clear after reconstitution | 96.7 | 95.2 |
| 2 | Before lyophilisation | Clear | 96.9 | 96.6 |
| | After lyophilisation | White cake | 96.7 | 96.1 |
| | 40°C D23 after lyophilisation | Clear after reconstitution | 96.6 | 95.6 |
| Notes: In the table, "D" indicates day, for example, D23 indicates 23 days. | | | | |

**Example 5: Screening of Protein Concentration in Anti-B7H3-ADC Antibody Formulations**

The 30 mM SA buffer system at pH 5.5 was used to prepare different protein concentrations of anti-B7H3-ADC formulations containing 40 mg/mL sucrose, 0.2 mg/mL PS80 and 7.6 mg/mL glycine. Specifically:

1) 20 mg/mL ADC-1
2) 50 mg/mL ADC-1
3) 60 mg/mL ADC-1
4) 70 mg/mL ADC-1

The prepared formulation was filtered, filled, sealed and capped. The samples were placed under shaking (25°C, 300 rpm, six days), freeze-thaw (at -35°C to 2 - 8°C for five cycles) and high temperature (40°C M1) conditions, and the stability of the formulation was investigated with the appearance, SEC and DAR values as evaluation indicators. The experimental results are shown in Table 7.

The experimental results showed that under the storage conditions, there were no significant differences in appearance, purity and toxin load among the 20 mg/mL ADC-1, 50 mg/mL ADC-1, 60 mg/mL ADC-1 and 70 mg/mL ADC-1 groups.

Table 7. Screening Experiment Results of Surfactant in Formulation

| Serial No. | Storage Conditions | Appearance | SEC Monomer% | DAR Value |
|---|---|---|---|---|
| 1 | D0 | Clear | 97.4 | 4.8 |
| | Shaking D6 | Clear | 97.1 | 4.8 |
| | FT 5Cycle | Clear | 97.2 | 4.8 |
| | 40°C M1 | Clear | 91.5 | 4.7 |
| 2 | D0 | Clear | 96.9 | 4.8 |
| | Shaking D6 | Clear | 97.1 | 4.8 |
| | FT 5Cycle | Clear | 97.0 | 4.8 |
| | 40°C M1 | Clear | 90.7 | 4.7 |
| 3 | D0 | Clear | 97.2 | 4.8 |
| | Shaking D6 | Clear | 97.1 | 4.8 |
| | FT 5Cycle | Clear | 96.9 | 4.8 |
| | 40°C M1 | Clear | 90.2 | 4.7 |
| 4 | D0 | Clear | 97.1 | 4.8 |
| | Shaking D6 | Clear | 97.0 | 4.8 |
| | FT 5Cycle | Clear | 96.9 | 4.8 |
| | 40°C M1 | Clear | 89.9 | 4.7 |
| Notes: In the table, "D" indicates day, for example, D0 indicates the start of the experiment; "M" indicates month, for example, M1 indicates one month; "FT 5Cycle" indicates five freeze-thaw cycles. | | | | |

**Example 6: Stability Experiment of Anti-B7H3-ADC Antibody Formulation**

An anti-B7H3-ADC formulation containing ADC-1 at a protein concentration of 20 mg/mL, 30 mM succinic acid-sodium succinate (SA) pH 5.5, 40 mg/mL sucrose, 7.6 mg/mL glycine and 0.2 mg/mL PS80 was prepared.

The prepared formulation was filtered, filled, sealed and capped. The stability of the samples under forced degradation conditions (-35°C to 2 - 8°C for five freeze-thaw cycles or shaking for eight days) and long-term storage conditions (2 - 8°C for 16 days) was investigated, and the stability of the formulation was investigated with the appearance, SEC and reduced R-CE as evaluation indicators. The experimental results are shown in Table 8.

The experimental results showed that from the appearance, the samples under various forced degradation conditions remained clear; there were no significant differences in purity and the stability was good.

Table 8. Stability Experiment Results of Formulation

| Serial No. | Storage Conditions | Appearance | SEC Monomer% | R-CE% |
|---|---|---|---|---|
| 1 | D0 | Clear | 96.4 | 96.0 |
| | Shaking for 8 days | Clear | 97.3 | 95.6 |
| | FT5C | Clear | 97.3 | 95.6 |
| | 2 - 8°C for 16 days | Clear | 97.3 | 95.6 |
| Notes: In the table, "D" indicates day, for example, D0 indicates the start of the experiment; FT5C indicates five freeze-thaw cycles. | | | | |

**Example 7: Formula Optimisation Experiment of B7H3-ADC Formulation**

Optimisation Experiment 1:

For the anti-B7H3-ADC formulation containing 7.6 mg/mL glycine and 40 mg/mL sucrose, a DOE (Design of Experiment) experiment was conducted with the pH value of 30 mM succinic acid-sodium succinate (SA), the protein concentration of ADC-1 and the concentration of PS80 as variables. DOE, experimental factors and levels were set as: pH 5 - 6, ADC-1 protein concentration of 10 - 30 mg/mL and PS80 concentration of 0.1 - 0.3 mg/mL, to design a series of formulae (see Table 9). The lyophilised samples underwent forced degradation experiments at a high temperature of 40°C for one month, and the evaluation indicators included appearance and SEC; see the results in Table 9.

The results indicated that the formulations of various formulae kept at a high temperature of 40°C for one month were clear, and had no significant differences in purity between SEC groups. Therefore, the B7H3-ADC samples exhibited good stability when the pH was 5 - 6, the protein concentration of ADC-1 was 10 - 30 mg/mL, and the concentration of PS80 was 0.1 - 0.3 mg/mL.

Table 9. Formula Design in DOE Formula Screening Experiment

| Serial No. | pH | Protein Concentration (mg/mL) | PS80 Concentration (mg/mL) |
|---|---|---|---|
| 1 | 5.5 | 20 | 0.2 |
| 2 | 5 | 10 | 0.1 |
| 3 | 6 | 10 | 0.3 |
| 4 | 6 | 10 | 0.1 |
| 5 | 5 | 30 | 0.2 |
| 6 | 6 | 30 | 0.1 |
| 7 | 5.5 | 20 | 0.1 |
| 8 | 6 | 20 | 0.221 |
| 9 | 5.5 | 10 | 0.2 |
| 10 | 5 | 15.5 | 0.3 |
| 11 | 5.5 | 30 | 0.3 |

Table 10. DOE Screening Experiment Results

| Serial No. | Storage Conditions | Appearance | SEC Monomer/% |
|---|---|---|---|
| 1 | D0 | Clear | 97.1 |
| | 40°C M1 | Clear | 97.1 |
| 2 | D0 | Clear | 97.0 |
| | 40°C M1 | Clear | 97.4 |
| 3 | D0 | Clear | 97.1 |
| | 40°C M1 | Clear | 97.4 |

(continued)

| Serial No. | Storage Conditions | Appearance | SEC Monomer/% |
|---|---|---|---|
| 4 | D0 | Clear | 97.2 |
| | 40°C M1 | Clear | 97.5 |
| 5 | D0 | Clear | 96.8 |
| | 40°C M1 | Clear | 97.1 |
| 6 | D0 | Clear | 97.2 |
| | 40°C M1 | Clear | 97.5 |
| 7 | D0 | Clear | 97.3 |
| | 40°C M1 | Clear | 97.3 |
| 8 | D0 | Clear | 97.0 |
| | 40°C M1 | Clear | 97.2 |
| 9 | D0 | Clear | 97.2 |
| | 40°C M1 | Clear | 97.4 |
| 10 | D0 | Clear | 97.0 |
| | 40°C M1 | Clear | 97.4 |
| 11 | D0 | Clear | 97.3 |
| | 40°C M1 | Clear | 97.5 |
| Notes: In the table, "D" indicates day, for example, D0 indicates the start of the experiment; "M" indicates month, for example, M1 indicates one month. | | | |

Optimisation Experiment 2:

Based on the anti-B7H3-ADC formulation containing ADC-1 at a protein concentration of 50 mg/mL, 40 mg/mL sucrose, 7.6 mg/mL glycine and 0.2 mg/mL PS80 in the 30 mM SA succinic acid-sodium succinate buffer at pH 5.5, a series of formulae were designed with the buffer concentration, pH, sucrose concentration, glycine concentration, PS80 concentration and ADC-1 protein concentration as variables (see Table 11). The lyophilised samples undergo forced degradation experiments at a high temperature of 40°C for one month, and the evaluation was performed with the appearance, SEC purity and DAR values as evaluation indicators; see the results in Table 12.

The results indicated that the formulations of various formulae kept at a high temperature of 40°C for one month were clear, and had no significant inter-group differences in SEC purity and DAR toxin load. Therefore, the B7H3-ADC samples exhibited good stability when the SA concentration was 20 - 30 mM, the pH was 5.0 - 5.5, the protein concentration of ADC-1 was 20 - 70 mg/mL, the sucrose concentration was 40 - 80 mg/mL, and the PS80 concentration was 0.2 - 0.8 mg/mL.

Table 11. Formula Design in DOE Formula Screening Experiment

| Serial No. | Buffer System and pH | Sucrose Concentration (mg/mL) | Glycine Concentration (mg/mL) | PS80 Concentration (mg/mL) | Protein Concentration (mg/mL) |
|---|---|---|---|---|---|
| 1 | 30mM SA | 40 | 7.6 | 0.2 | 20 |
| 2 | 30mM SA | 40 | 7.6 | 0.2 | 50 |
| 3 | 30mM SA pH | 40 | 7.6 | 0.2 | 50 |
| 4 | 30mM SA pH | 40 | 7.6 | 0.2 | 60 |
| 5 | 30mM SA | 40 | 7.6 | 0.2 | 70 |
| 6 | 30mM SA | 40 | 7.6 | 0.4 | 50 |
| 7 | 30mM SA | 40 | 7.6 | 0.6 | 50 |

(continued)

| Serial No. | Buffer System and pH | Sucrose Concentration (mg/mL) | Glycine Concentration (mg/mL) | PS80 Concentration (mg/mL) | Protein Concentration (mg/mL) |
|---|---|---|---|---|---|
| 8 | 30mM SA | 40 | 7.6 | 0.8 | 50 |
| 9 | 20mM SA | 80 | 0 | 0.2 | 50 |
| 10 | 30mM SA | 80 | 0 | 0.2 | 50 |

Table 12. DOE Screening Experiment Results

| Serial No. | Storage Conditions | Appearance | SEC Monomer% | DAR Value |
|---|---|---|---|---|
| 1 | D0 | Clear | 98.24 | 3.6 |
| | 40°C W4 | Clear | 97.73 | 3.5 |
| 2 | D0 | Clear | 98.16 | 3.6 |
| | 40°C W4 | Clear | 97.15 | 3.5 |
| 3 | D0 | Clear | 98.25 | 3.6 |
| | 40°C W4 | Clear | 97.11 | 3.5 |
| 4 | D0 | Clear | 98.13 | 3.6 |
| | 40°C W4 | Clear | 96.92 | 3.5 |
| 5 | D0 | Clear | 98.10 | 3.6 |
| | 40°C W4 | Clear | 96.87 | 3.5 |
| 6 | D0 | Clear | 98.19 | 3.6 |
| | 40°C W4 | Clear | 97.10 | 3.5 |
| 7 | D0 | Clear | 98.24 | 3.6 |
| | 40°C W4 | Clear | 97.15 | 3.5 |
| 8 | D0 | Clear | 98.16 | 3.6 |
| | 40°C W4 | Clear | 97.17 | 3.5 |
| 9 | D0 | Clear | 98.25 | 3.6 |
| | 40°C W4 | Clear | 97.59 | 3.5 |
| 10 | D0 | Clear | 98.17 | 3.6 |
| | 40°C W4 | Clear | 97.57 | 3.5 |
| Notes: In the table, "D" indicates day, "W" indicates week, for example, D0 indicates the start of the experiment and W4 indicates four weeks. | | | | |

**Example 8: Lyophilisation of Anti-B7H3-ADC Antibody Formulation**

An anti-B7H3-ADC antibody formulation containing ADC-1 at a protein concentration of 20 mg/mL, 40 mg/mL sucrose, 7.6 mg/mL glycine and 0.2 mg/mL PS80 was prepared in the 30 mM SA succinic acid-sodium succinate buffer at pH 5.5. The formulation samples were lyophilised using a lyophilisation procedure consisting of pre-freezing, primary drying and secondary drying; see the lyophilisation procedure in Table 13. After the end of the lyophilisation procedure, vacuum sealing was performed. The samples before and after lyophilisation were evaluated with the appearance, SEC and R-CE as evaluation indicators; see the results in Table 14. The results indicated that the reconstituted solution could maintain good performance of the liquid formulation.

Table 13. Lyophilisation Procedure

| Lyophilisation Process Parameters | Set Temperature (°C) | Set Time (Minutes) | Hold Time (Minutes) | Vacuum Degree (Pa) |
|---|---|---|---|---|
| Pre-freezing | 5 | 10 | 60 | N/A |
| Pre-freezing | -45 | 50 | 120 | N/A |
| Primary Drying | -20 | 120 | 3000 | 10 |
| Secondary Drying | 25 | 60 | 1 | 10 |
| | 25 | 1 | 600 | 1 |

Table 14. Experimental Results Before and After Lyophilisation

| Serial No. | Storage Conditions | Appearance | SEC Monomer% |
|---|---|---|---|
| 1 | Before lyophilisation | Clear | 96.9 |
| | After lyophilisation | White cake, clear after reconstitution | 96.7 |

## Claims

1. A pharmaceutical composition comprising an antibody-drug conjugate and a buffer, **characterised in that** the antibody-drug conjugate has the structure shown as follows:

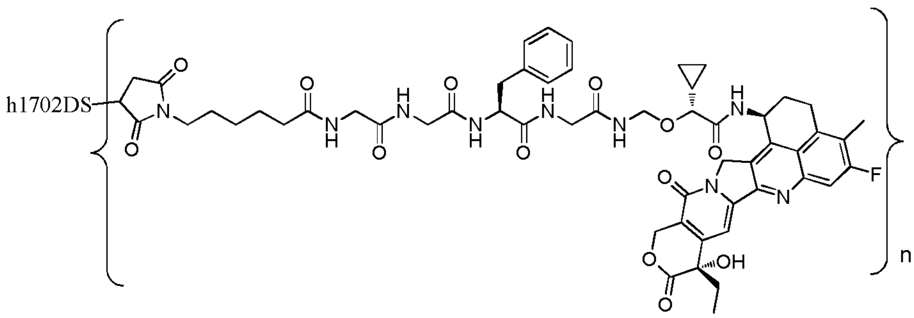

;

Wherein:

h1702DS is an anti-B7H3 antibody comprising a heavy chain as shown in SEQ ID NO: 1 and a light chain as shown in SEQ ID NO:2;
n ranges from 1 to 10, preferably 1 to 8, more preferably 3 to 5, and further more preferably about 4;
The buffer for the pharmaceutical composition is succinate buffer or histidine buffer, preferably succinic acid-sodium succinate or histidine-acetate.

2. The pharmaceutical composition according to Claim 1, **characterised in that** the concentration of the buffer is about 10 mM to about 50 mM, preferably about 20 mM to about 40 mM, and more preferably about 30 mM.

3. The pharmaceutical composition according to Claim 1 or 2, **characterised in that** its pH is about 4.5 to 6.5, preferably about 5.0 to 6.0 and more preferably about 5.5 to 5.6.

4. The pharmaceutical composition according to any one of Claims 1 to 3, **characterised in that** it further comprises a surfactant, which is preferably polysorbate, more preferably polysorbate 80 or polysorbate 20, and most preferably polysorbate 80.

5. The pharmaceutical composition according to Claim 4, **characterised in that** the concentration of the surfactant is about 0.01 mg/mL to about 1.0 mg/mL, preferably about 0.2 mg/mL to about 0.8 mg/mL, and more preferably about 0.2 mg/mL.

6. The pharmaceutical composition according to any one of Claims 1 to 5, **characterised in that** it further comprises a sugar, which is preferably sucrose, mannitol or trehalose, and more preferably sucrose or trehalose.

7. The pharmaceutical composition according to Claim 6, **characterised in that** the concentration of the sugar is about 25 mg/mL to about 80 mg/mL, preferably about 40 mg/mL to about 60 mg/mL, and more preferably about 40 mg/mL.

8. The pharmaceutical composition according to any one of Claims 1 to 7, **characterised in that** it further comprises an amino acid or an amino acid salt, which is glycine or arginine hydrochloride, and preferably glycine.

9. The pharmaceutical composition according to Claim 8, **characterised in that** the concentration of the amino acid or amino acid salt is about 5 mg/mL to about 10 mg/mL, preferably about 7 mg/mL to about 8 mg/mL, and more preferably about 7.6 mg/mL.

10. The pharmaceutical composition according to any one of Claims 1 to 9, **characterised in that** the concentration of the antibody-drug conjugate is measured by protein concentration, which ranges from about 10 mg/mL to about 70 mg/mL, preferably about 20 mg/mL to about 50 mg/mL, more preferably about 20 mg/mL to about 30 mg/mL or about 30 mg/mL to about 50 mg/mL, and further preferably about 20 mg/mL or about 30 mg/mL or about 50 mg/mL.

11. The pharmaceutical composition according to any one of Claims 1 to 10, **characterised in that** it comprises the following components:
(a) the antibody-drug conjugate at a protein concentration of about 20 mg/mL to about 70 mg/mL, (b) polysorbate at about 0.2 mg/mL to about 0.8 mg/mL, (c) sugar at about 25 mg/mL to about 80 mg/mL, (d) glycine at about 7 mg/mL to about 8 mg/mL, and (e) succinate buffer at about 10 mM to about 40 mM; the pH of the composition is about 5.0 - 6.0.

Preferably, the pharmaceutical composition comprises the following components:
(a) the antibody-drug conjugate at a protein concentration of about 20 mg/mL to about 50 mg/mL, (b) polysorbate 80 at about 0.2 mg/mL to about 0.8 mg/mL, (c) sucrose at about 40 mg/mL to about 80 mg/mL, (d) glycine at about 7.6 mg/mL, and (e) succinic acid-sodium succinate at about 30 mM; the pH of the composition is about 5.5 - 5.6;
More preferably, the pharmaceutical composition comprises the following components:

(a) the antibody-drug conjugate at a protein concentration of about 20 mg/mL to about 30 mg/mL, (b) polysorbate 80 at about 0.2 mg/mL, (c) sucrose at about 40 mg/mL, (d) glycine at about 7.6 mg/mL, and (e) succinic acid-sodium succinate at about 30 mM; the pH of the composition is about 5.5 - 5.6;
(a) the antibody-drug conjugate at a protein concentration of about 30 mg/mL to about 50 mg/mL, (b) polysorbate 80 at about 0.2 mg/mL, (c) sucrose at about 40 mg/mL, (d) glycine at about 7.6 mg/mL, and (e) succinic acid-sodium succinate at about 30 mM; the pH of the composition is about 5.5 - 5.6.

12. A lyophilised formulation containing the antibody-drug conjugate, **characterised in that** it forms the pharmaceutical composition described in any one of Claims 1 to 11 upon reconstitution.

13. A method to prepare the lyophilised formulation containing the antibody-drug conjugate, **characterised in that** it comprises the step to lyophilise the pharmaceutical composition described in any one of Claims 1 to 11.

14. A lyophilised formulation containing the antibody-drug conjugate, **characterised in that** it is obtained by lyophilising the pharmaceutical composition described in any one of Claims 1 to 11.

15. A reconstituted solution containing the antibody-drug conjugate, **characterised in that** it is obtained by reconstituting the lyophilised formulation described in Claim 12 or 14; the reconstituted solution comprises the following components:
(a) the antibody-drug conjugate at a protein concentration of about 20 mg/mL to about 70 mg/mL, (b) polysorbate at about 0.2 mg/mL to about 0.8 mg/mL, (c) sugar at about 25 mg/mL to about 80 mg/mL, (d) glycine at about 7 mg/mL to about 8 mg/mL, and (e) succinate buffer at about 10 mM to about 40 mM; the pH of the composition is about 5.0 - 6.0.

Preferably, the reconstituted solution comprises the following components:
(a) the antibody-drug conjugate at a protein concentration of about 20 mg/mL to about 50 mg/mL, (b) polysorbate 80 at about 0.2 mg/mL to about 0.8 mg/mL, (c) sucrose at about 40 mg/mL to about 80 mg/mL, (d) glycine at about 7.6 mg/mL, and (e) succinic acid-sodium succinate at about 30 mM; the pH of the composition is about 5.5 - 5.6;
More preferably, the reconstituted solution comprises the following components:

(a) the antibody-drug conjugate at a protein concentration of about 20 mg/mL to about 30 mg/mL, (b) polysorbate 80 at about 0.2 mg/mL, (c) sucrose at about 40 mg/mL, (d) glycine at about 7.6 mg/mL, and (e) succinic acid-sodium succinate at about 30 mM; the pH of the composition is about 5.5 - 5.6;
(a) the antibody-drug conjugate at a protein concentration of about 30 mg/mL to about 50 mg/mL, (b) polysorbate 80 at about 0.2 mg/mL, (c) sucrose at about 40 mg/mL, (d) glycine at about 7.6 mg/mL, and (e) succinic acid-sodium succinate at about 30 mM; the pH of the composition is about 5.5 - 5.6.

**16.** A product, **characterised in that** it comprises a container containing the pharmaceutical composition described in any one of Claims 1 to 11, the lyophilised formulation described in Claim 12 or 14 or the reconstituted solution described in Claim 15.

**17.** The use of the pharmaceutical composition described in any one of Claims 1 to 11, the lyophilised formulation described in Claim 12 or 14, the reconstituted solution described in Claim 15 or the product described in Claim 16 in the preparation of a medication for the treatment of diseases; preferably, the disease is B7H3 expression related cancer; more preferably, the cancer is breast cancer, ovarian cancer, cervical cancer, uterine cancer, prostate cancer, kidney cancer, urethral cancer, bladder cancer, liver cancer, gastric cancer, endometrial cancer, salivary gland cancer, esophagoeal cancer, melanoma, glioma, neuroblastoma, sarcoma, lung cancer, colon cancer, rectal cancer, colorectal cancer, leukaemia, bone cancer, skin cancer, thyroid cancer, pancreatic cancer or lymphoma.

# INTERNATIONAL SEARCH REPORT

International application No.
**PCT/CN2023/100483**

**A. CLASSIFICATION OF SUBJECT MATTER**

A61K39/395(2006.01)i; A61K47/68(2017.01)i; A61K9/19(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pubmed, STN on web, genbank, NCBI, 中文专利序列检索系统, Chinese Patent Sequence Search System: 上海翰森生物医药科技有限公司; 常州恒邦药业有限公司; 江苏豪森药业集团有限公司, 毛东杰, 偶联物, conjugate, 依喜替康, exatecan, B7H3, h1702, 缓冲剂, PBS, buffer, 琥珀酸, succinic, 聚山梨酯, 吐温, tween, 糖, 蔗糖, sugar, 甘氨酸, Ala, SEQ ID NOs: 1-2

**C. DOCUMENTS CONSIDERED TO BE RELEVANT**

| Category* | Citation of document, with indication, where appropriate, of the relevant passages | Relevant to claim No. |
|---|---|---|
| Y | WO 2021190581 A1 (JIANGSU HENGRUI PHARMACEUTICALS CO., LTD. et al.) 30 September 2021 (2021-09-30)<br>description, abstract, and claims 1-11 and 14-19 | 1-17 |
| Y | WO 2020063673 A1 (JIANGSU HENGRUI PHARMACEUTICALS CO., LTD. et al.) 02 April 2020 (2020-04-02)<br>description, page 44, paragraphs 3-10, and page 46, embodiment 11 | 1-17 |
| Y | WO 2021190586 A1 (JIANGSU HENGRUI PHARMACEUTICALS CO., LTD. et al.) 30 September 2021 (2021-09-30)<br>description, page 28, lines 10-29, page 51, line 20-page 52, line 9 | 1-17 |
| Y | CN 101237881 A (IMMUNOGEN INC.) 06 August 2008 (2008-08-06)<br>description, abstract, and claims 1 and 4 | 8-17 |
| A | WO 2021136274 A1 (BIO-THERA SOLUTIONS, LTD.) 08 July 2021 (2021-07-08)<br>entire document | 1-17 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 September 2023** | **20 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/100483**

**C. DOCUMENTS CONSIDERED TO BE RELEVANT**

| Category* | Citation of document, with indication, where appropriate, of the relevant passages | Relevant to claim No. |
|---|---|---|
| A | WO 2020233534 A1 (BIO-THERA SOLUTIONS, LTD.) 26 November 2020 (2020-11-26) entire document | 1-17 |
| A | CN 105163763 A (ABBVIE DEUTSCHLAND GMBH & CO. KG et al.) 16 December 2015 (2015-12-16) entire document | 1-17 |

Form PCT/ISA/210 (second sheet) (July 2022)

**INTERNATIONAL SEARCH REPORT**
**Information on patent family members**

International application No.
**PCT/CN2023/100483**

| Patent document cited in search report | | | Publication date (day/month/year) | Patent family member(s) | | | Publication date (day/month/year) |
|---|---|---|---|---|---|---|---|
| WO | 2021190581 | A1 | 30 September 2021 | BR | 112022019073 | A2 | 08 November 2022 |
| | | | | KR | 20220157998 | A | 29 November 2022 |
| | | | | JP | 2023518583 | A | 02 May 2023 |
| | | | | AU | 2021243073 | A1 | 13 October 2022 |
| | | | | TW | 202144013 | A | 01 December 2021 |
| | | | | US | 2023165969 | A1 | 01 June 2023 |
| | | | | EP | 4129345 | A1 | 08 February 2023 |
| | | | | CA | 3175733 | A1 | 30 September 2021 |
| WO | 2020063673 | A1 | 02 April 2020 | MX | 2021003446 | A | 15 June 2021 |
| | | | | JP | 2022512568 | A | 07 February 2022 |
| | | | | EP | 3854816 | A1 | 28 July 2021 |
| | | | | EP | 3854816 | A4 | 07 September 2022 |
| | | | | TW | 202028242 | A | 01 August 2020 |
| | | | | CA | 3114474 | A1 | 02 April 2020 |
| | | | | AU | 2019351427 | A1 | 15 April 2021 |
| | | | | BR | 112021004829 | A2 | 08 June 2021 |
| | | | | KR | 20210068457 | A | 09 June 2021 |
| | | | | US | 2021347894 | A1 | 11 November 2021 |
| WO | 2021190586 | A1 | 30 September 2021 | TW | 202144015 | A | 01 December 2021 |
| CN | 101237881 | A | 06 August 2008 | ZA | 200800146 | A | 28 October 2009 |
| | | | | ZA | 200800146 | A | 28 October 2009 |
| WO | 2021136274 | A1 | 08 July 2021 | | None | | |
| WO | 2020233534 | A1 | 26 November 2020 | | None | | |
| CN | 105163763 | A | 16 December 2015 | RU | 2015144287 | A | 24 April 2017 |
| | | | | RU | 2015144287 | A3 | 19 March 2018 |
| | | | | NZ | 630826 | A | 30 June 2017 |
| | | | | RU | 2020125266 | A | 24 September 2020 |
| | | | | JP | 2016515511 | A | 30 May 2016 |
| | | | | JP | 6382935 | B2 | 29 August 2018 |
| | | | | ES | 2727134 | T3 | 14 October 2019 |
| | | | | NZ | 732015 | A | 28 January 2022 |
| | | | | AU | 2020277164 | A1 | 24 December 2020 |
| | | | | EP | 2968588 | A1 | 20 January 2016 |
| | | | | EP | 2968588 | B1 | 09 January 2019 |
| | | | | TW | 202146054 | A | 16 December 2021 |
| | | | | KR | 20150131366 | A | 24 November 2015 |
| | | | | KR | 102305226 | B1 | 29 September 2021 |
| | | | | JP | 2018184468 | A | 22 November 2018 |
| | | | | KR | 20220139443 | A | 14 October 2022 |
| | | | | CA | 2906130 | A1 | 18 September 2014 |
| | | | | KR | 20210118238 | A | 29 September 2021 |
| | | | | US | 2014286969 | A1 | 25 September 2014 |
| | | | | RS | 58666 | B1 | 28 June 2019 |
| | | | | TW | 201945032 | A | 01 December 2019 |
| | | | | SG | 11201507430 | RA | 29 October 2015 |
| | | | | EP | 3517132 | A1 | 31 July 2019 |
| | | | | SG | 10201707633 | VA | 29 November 2017 |
| | | | | ME | 03457 | B | 20 January 2020 |
| | | | | AU | 2018267662 | A1 | 13 December 2018 |

Form PCT/ISA/210 (patent family annex) (July 2022)

**INTERNATIONAL SEARCH REPORT**
**Information on patent family members**

International application No.
**PCT/CN2023/100483**

| Patent document cited in search report | Publication date (day/month/year) | Patent family member(s) | | | Publication date (day/month/year) |
|---|---|---|---|---|---|
| | | HK | 1219044 | A1 | 24 March 2017 |
| | | JP | 2020143152 | A | 10 September 2020 |
| | | AU | 2014228172 | A1 | 24 September 2015 |
| | | AU | 2014228172 | B2 | 06 December 2018 |
| | | KR | 20220041957 | A | 01 April 2022 |
| | | MX | 2020000333 | A | 13 July 2020 |
| | | PT | 2968588 | T | 08 May 2019 |
| | | BR | 112015023391 | A2 | 28 November 2017 |
| | | BR | 112015023391 | A8 | 30 January 2018 |
| | | WO | 2014143765 | A1 | 18 September 2014 |
| | | WO | 2014143765 | A8 | 11 September 2015 |
| | | SI | 2968588 | T1 | 31 May 2019 |
| | | LT | 2968588 | T | 10 May 2019 |
| | | AU | 2023200432 | A1 | 02 March 2023 |
| | | MX | 2015012549 | A | 21 June 2016 |
| | | DK | 2968588 | T3 | 23 April 2019 |
| | | HK | 1217644 | A1 | 20 January 2017 |
| | | TW | 201519903 | A | 01 June 2015 |
| | | TWI | 689313 | B | 01 April 2020 |
| | | TR | 201905313 | T4 | 21 May 2019 |
| | | IL | 274881 | A | 30 July 2020 |
| | | PL | 2968588 | T3 | 30 August 2019 |
| | | IL | 241009 | A0 | 30 November 2015 |
| | | JP | 2022126754 | A | 30 August 2022 |
| | | HUE | 044071 | T2 | 30 September 2019 |
| | | HRP | 20190672 | T1 | 23 August 2019 |
| | | US | 2020282072 | A1 | 10 September 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014057687 A
- WO 2020063673 A1
- WO 2020063673 A
- US 20050238649 A1
- US 5208020 A


### Non-patent literature cited in the description


- **MULLARD A**. *Nature Reviews Drug Discovery*, 2013, vol. 12, 329-332
- **DIJOSEPH JF** ; **ARMELLINO DC**. *Blood*, 2004, vol. 103, 1807-1814
- *Clinical Cancer Research*, 2016, vol. 22 (20), 5097-5108
- *Cancer Sci*, 2016, vol. 107, 1039-1046
- **CHARI et al.** *Cancer Research*, 1992, vol. 52, 127-131
- *J. biol. chem*, 1968, vol. 243, 3558
- Antibody Experimental Manual. Cold Spring Harbor Laboratory
- Molecular Cloning Manual. Cold Spring Harbor Laboratory